(19) Europäisches Patentamt European Patent Office Office européen des brevets

(11) **EP 1 783 480 B1**

(12) **FASCICULE DE BREVET EUROPEEN**

(45) Date de publication et mention
de la délivrance du brevet:
**23.07.2008 Bulletin 2008/30**

(51) Int Cl.:
***G01N 21/35*** *(2006.01)*     ***G01N 21/27*** *(2006.01)*
***G01N 33/28*** *(2006.01)*

(21) Numéro de dépôt: **06291546.7**

(22) Date de dépôt: **02.10.2006**

(54) **Méthode de détermination de la teneur en diolefines conjuguees d'un echantillon a partir de son spectre proche infrarouge et application au controle d'unités**

Verfahren zur Feststellung des konjugierten Diolefinenehaltes einer Probe mittels eines Spektrums in Nahinfraroten Bereich und Verfahren zur Steuerung einer Einheit

Method for the determination of the conjugated diolefins content of a sample using near infrared spectrum and method for controlling a unit

(84) Etats contractants désignés:
**BE DE FR GB NL**

(30) Priorité: **04.11.2005 FR 0511277**

(43) Date de publication de la demande:
**09.05.2007 Bulletin 2007/19**

(73) Titulaire: **Institut Français du Pétrole
92852 Rueil Malmaison Cédex (FR)**

(72) Inventeurs:
• **Picard Florent
  69360 Communay (FR)**
• **Lopez Garcia Clementina
  69008 Lyon (FR)**

• **Bader, Jean-Marc
  69440 Taluyers (FR)**
• **Wahl Francois
  69630 Champonost (FR)**

(56) Documents cités:
EP-A- 0 801 299     WO-A-97/14951
FR-A- 2 619 625     US-A- 5 452 232
US-B1- 6 281 499

• ZANIER-SZYDLOWSKI N ET AL: "CONTROL OF REFINING PROCESSES ON MID-DISTILLATES BY NEAR INFRARED SPECTROSCOPY" REVUE DE L'INSTITUT FRANCAIS DU PETROLE, EDITIONS TECHNIP. PARIS, FR, vol. 54, no. 4, juillet 1999 (1999-07), pages 463-472, XP000873938 ISSN: 1294-4475

**Description**

DOMAINE DE L'INVENTION

L'invention concerne le domaine des méthodes de détermination de diverses propriétés de coupes hydrocarbures, en particulier les essences, et plus particulièrement les essences de craquage catalytique, les essences de vapocraquage, les essences de cokéfaction et leurs mélanges, à partir de leur spectre obtenu dans le Proche Infrarouge (noté spectre PIR en abrégé). Cette invention s'applique de manière générale à des essences qui contiennent du soufre (entre 10 ppm et 10000 ppm), des oléfines (au moins 5 % poids) et des dioléfines (au moins 0,1 % poids). Le point final de distillation de ces essences doit être inférieur à 300°C, et de préférence 250°C.

Le domaine du proche infra rouge correspond à des longueurs d'onde comprises approximativement entre 800 nanomètres et 2500 nanomètres (14286 à 4000 cm$^{-1}$). Les propriétés déterminables à partir du spectre PIR des essences sont très diverses, les principales étant jusqu'à maintenant la densité, les indices d'octane (RON et MON), la tension de vapeur Reid (RVP), la distillations ASTM, et la teneur en familles chimiques (paraffines, aromatiques, naphtènes, oléfines) entre autres.

Dans la présente invention la propriété dont il est question est la teneur en dioléfines conjuguées.

Cette teneur est indirectement mesurée par des méthodes chimiques qui font appel à la réaction de Diels Alder. Le principe de la méthode est l'addition stoechiométrique de l'anhydride maléique sur les dioléfines conjuguées contenues dans l'échantillon. Les méthodes connues qui s'appuient sur cette réaction sont par exemple la DV abréviation dans la terminologie anglo saxonne de "diene value" ["Laboratory Test Methods for Petroleum and its Products", Universal Oil Products Method N° 326-82, Universal Oil Products, Illinois, USA.] ou bien la MAV, abréviation de "maleic anhydrid value" en anglais.

Dans ce brevet nous ferons référence à la MAV.

La MAV est exprimée en nombre de milligrammes d'anhydride maléique qui réagissent avec un gramme d'échantillon. Cette mesure est proportionnelle à la teneur en dioléfines conjuguées présentes dans l'échantillon analysé.

La détermination de la teneur en dioléfines conjuguées d'un échantillon d'essence par analyse chimique selon la réaction de Diels Alder est une opération relativement longue, de l'ordre de 5 à 7 heures par analyse. En outre, cette analyse est potentiellement dangereuse car elle nécessite le chauffage du mélange d'un solvant tel que le toluène avec l'essence, et la manipulation d'autres solvants tels que des éthers.

La détermination de la teneur en dioléfines conjuguées par la méthode Diels-Alder est facultativement utilisée pour mesurer les performances des unités industrielles d'hydrogénation sélective des essences craquées, de préférence des essences issues des unités de craquage catalytique, des unités de vapocraquage et des unités de cokéfaction. Toutefois, du fait des opérations nécessaires à l'analyse telles que des extractions liquide ou des transvasements de produits, il n'est pas envisageable d'automatiser la méthode pour en faire une méthode de contrôle en continu des unités industrielles d'hydrogénation sélective.

C'est un des objets de la présente invention de présenter une nouvelle méthode de détermination de la teneur en dioléfines conjuguées dans les essences par l'intermédiaire de la mesure de la MAV ou de la variation de la MAV à partir du spectre PIR, à la fois plus rapide et plus répétable que la méthode de détermination par analyse chimique, et d'utiliser la dite nouvelle méthode dans une chaîne de contrôle et régulation des unités industrielles d'hydrogénation sélective des essences.

**EXAMEN DE L'ART ANTERIEUR**

L'art antérieur est représenté par des brevets et diverses communications décrivant des méthodes de détermination d'une grande variété de propriétés telles que la densité, les indices d'octane (RON et MON), la tension de vapeur Reid (RVP), la distillations ASTM, la teneur en familles chimiques (paraffines, aromatiques, naphtènes, oléfines) et la MAV à partir d'un spectre PIR.

Parmi les brevets de ce domaine, on peut retenir le brevet US 6,070,128 [2000] comme décrivant l'état de l'art le plus proche du point de vue de la méthode elle même.

Ce brevet décrit une méthode de détermination d'une propriété P d'une coupe hydrocarbure faisant appel à l'absorption d'un échantillon de la coupe considérée dans une zone de longueur d'onde comprise entre 600 et 2600 nanomètres (16667 à 3847 cm$^{-1}$) consistant à comparer le spectre obtenu avec les spectres d'un certain nombre d'échantillons formant une base pour lesquels la propriété P est connue.

La propriété P de l'échantillon considéré est obtenue par une méthode dite de topologie. Comme il sera exposé en détail plus loin, la méthode utilisée dans la présente invention pour déterminer la propriété P à partir du spectre PIR, en l'occurrence la MAV, est substantiellement différente de la méthode exposée dans le brevet US 6,070,128 cité puisque c'est la notion de différence de spectre entre le spectre d'un échantillon utilisé comme référence, et les spectres d'autres éléments de la base qui est utilisée.

Il en résulte un niveau de précision dans la prédiction de la MAV qui n'est pas accessible avec une méthode reposant sur la comparaison directe des spectres de l'échantillon étudié avec les spectres des éléments d'une base de propriété P connue.

Concernant les méthodes de contrôle et régulation des unités industrielles d'hydrogénation sélective, l'homme du métier connaît une méthode consistant à suivre la température du ou des lits catalytiques en entrée et en sortie, et à utiliser la différence de température entre l'entrée et la sortie du ou des lits comme paramètre de contrôle, c'est-à-dire, à fixer une valeur du delta T maximal admissible à partir de laquelle un des paramètres d'entrée de l'unité, le plus souvent la quantité d'hydrogène introduite, doit être modifié.

La valeur de la quantité d'hydrogène introduite dans l'unité est déterminée à partir d'un rapport arbitrairement fixé par rapport au débit de charge entrante, et d'un certain excès par rapport à la quantité d'hydrogène consommé par les réactions d'hydrogénation sélective des dioléfines et des oléfines.

La mesure de la MAV n'intervient donc que pour contrôler les réglages de l'unité a posteriori, mais aucunement pour assurer un réglage continu des paramètres de l'unité.

Une méthode de contrôle et régulation d'unités industrielles d'hydrogénation sélective de coupes essences oléfiniques correspondant au préambule de la revendication 1 est décrite dans le document FR 2 619 625.

C'est un des apports de la méthode décrite dans la présente invention que d'introduire une mesure continue et rapide de la teneur en dioléfines de l'essence à traiter (appelée charge) et de l'essence traitée (appelée effluent) dans le but de déterminer la quantité d'hydrogène à introduire dans l'unité pour se situer en continu au plus près de la stoechiométrie.

Une autre application de la mesure en continu de la MAV est de permettre l'ajustement de la température du ou des réacteur(s) pour assurer une conversion des dioléfines la plus proche possible de la cible fixée par l'opérateur.

Le fait de se situer en continu au plus près de la stoechiométrie a un impact important sur la quantité d'hydrogène consommé et donc sur l'économie du procédé.

## DESCRIPTION SOMMAIRE DES FIGURES

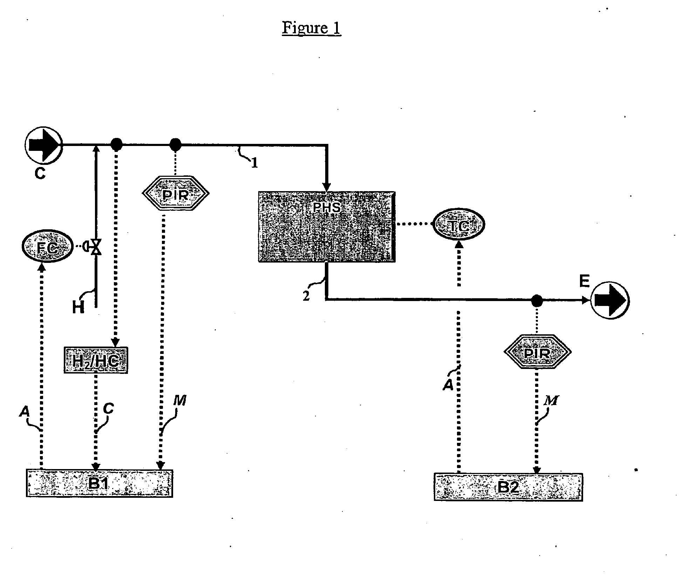

La figure 1 représente une vue schématique d'une unité d'hydrogénation sélective notée PHS sur laquelle on a représenté le système de contrôle et régulation objet de l'invention sous forme de deux boucles, notées B1 pour la première boucle et B2 pour la seconde boucle.

La signification des notations apparaissant sur la figure 1 est la suivante:

A : action
M : mesure
C : consigne
FC : contrôle de débit
TC : contrôle de température
PIR : mesure par proche infrarouge

H2/HC: rapport de débit d'hydrogène sur le débit de charge

La charge hydrocarbure à traiter (C) est introduite dans l'unité d'hydrogénation sélective (PHS) par la ligne (1), de laquelle est extrait l'effluent hydrogéné (E) par la ligne (2).

Les autres éléments de la figure 1 sont décrits en détail dans la description détaillée de l'invention.

## DESCRIPTION SOMMAIRE DE L'INVENTION

La description qui suit est donnée en référence à la MAV.

La MAV est une méthode de mesure des dioléfines conjuguées présentes dans une essence, basée sur l'addition stoechiométrique des dioléfines sur l'anhydride maléique. Elle s'exprime en nombre de milligrammes d'anhydride maléique ayant réagi sur un gramme d'échantillon.

La méthode décrite dans la présente invention est une méthode de détermination de la MAV d'essence de craquage catalytique ou d'essence de pyrolyse ou d'essence de cokéfaction ou d'un mélange quelconque des dites essences, à partir du spectre d'absorption obtenu dans le proche infra rouge (PIR) de la dite essence ou du dit mélange, c'est à dire dans une gamme de longueur d'ondes comprise entre 800 et 2500 nanomètres.

La méthode d'obtention de la MAV par spectre PIR fait appel à une comparaison selon un critère précis entre le spectre de l'échantillon étudié et les spectres d'un ensemble d'éléments formant une base dite base de calibration.

La base de calibration est constituée d'un ensemble de N échantillons pour chacun desquels on connaît à la fois le spectre PIR et la valeur de la MAV déterminée par la voie chimique.

La corrélation entre la MAV et le spectre PIR est établie à partir des couples de valeurs spectre PIR/MAV pour chacun des éléments de la base. Plus précisément, la corrélation est établie à partir des différences entre les spectres des éléments de la base, un élément de la base étant choisi comme élément de référence, et les différences entre les valeurs de la MAV pour l'échantillon étudié et pour l'échantillon de référence.

Ce point est très important car il permet de s'affranchir des différences dans la valeur de la MAV dues aux familles chimiques qui varient très peu au cours du procédé d'hydrogénation sélective (essentiellement les paraffines, les naphtènes, les aromatiques et les monooléfines) de manière à faire ressortir sélectivement les différences de la MAV dues précisément à la variation des dioléfines.

La méthode consiste à disposer d'une série d'échantillons de MAV inconnue, prise en entrée ou en sortie de l'unité, et à former les différences entre ces échantillons par rapport à un échantillon de la série pris comme référence. Former les différences entre échantillons signifie établir la différence entre les spectres des dits échantillons par rapport à un échantillon de référence pris à l'intérieur de la série.

C'est la différence de spectre qui est utilisée pour entrer dans la corrélation PIR issue de la base de calibration et qui permet de récupérer une valeur de différence de MAV.

Connaissant la valeur de la MAV de l'échantillon pris en référence, déterminée par voie chimique, on peut alors en déduire la valeur de la MAV pour les autres échantillons de la série.

La méthode qu'on appellera par la suite méthode "delta", comprend les étapes suivantes:

a) on choisit un échantillon prélevé dans une série qui sera utilisé comme échantillon de référence,

b) on détermine le spectre PIR ainsi que la MAV par la méthode chimique de l'échantillon de référence, c) on détermine le spectre PIR des autres éléments de la série,

d) on calcule la différence entre le spectre de l'échantillon de référence et celui d'un échantillon de la série dont la MAV est inconnue, cette différence de spectre s'appellant spectre soustrait

e) on vérifie que le spectre soustrait se trouve à l'intérieur du domaine du modèle PIR,

f) on calcule une valeur de variation de la MAV à partir du spectre soustrait en utilisant la corrélation issue de la base de calibration,

g) on calcule la MAV de l'échantillon étudié à partir de la variation de MAV obtenue à l'étape f) et de la MAV chimique de l'échantillon de référence.

Cette méthode peut être utilisée de manière séquentielle ou continue.

L'application de la méthode au contrôle et à la régulation des unités d'hydrogénation sélective se fait par deux boucles de régulation notée boucle 1 ( ou boucle d'entrée), et boucle 2 ( ou boucle de sortie) qui seront exposées en détail plus loin.

La détermination de la MAV de la charge d'une part, et de l'effluent d'autre part, est réalisée à partir d'une série d'échantillons prélevés au cours du temps selon la méthode précédemment décrite, l'échantillon de référence étant généralement le premier échantillon obtenu au cours du temps.

La fréquence de prélèvement est généralement d'un échantillon par heure, et préférentiellement d'un échantillon par demi heure.

Pour simplifier, on appellera dans la suite MAV d'entrée la MAV de la charge déterminée par la méthode "delta", et MAV de sortie la MAV de l'effluent déterminée par la méthode "delta".

Les deux boucles de régulation fonctionnent de manière simultanée et indépendante. Plus précisément, la méthode décrite dans la présente invention est une méthode de contrôle et régulation des unités d'hydrogénation sélective de coupes essences oléfiniques consistant en la succession d'étapes suivantes :

- on prélève un échantillon en entrée et en sortie de la dite unité à une fréquence déterminée,
- on analyse les dits échantillons par leur spectre PIR en absorption dans une gamme de longueur d'onde comprise entre 800 nm et 2500 nm,
- on détermine la $MAV_{PIR}$ des échantillons d'entrée et de sortie par un traitement des spectres PIR faisant appel à une base de calibration et un prétraitement spécifique,
- on détermine à partir de la $MAV_{PIR}$ de l'échantillon d'entrée et du débit de charge, la quantité d'hydrogène à introduire dans l'unité qui est utilisée comme valeur de consigne dans une première boucle de régulation de la dite valeur de la $MAV_{PIR}$ en entrée,
- on détermine à partir de la $MAV_{PIR}$ de l'échantillon de sortie et d'un modèle dynamique, la température d'entrée optimale dans le réacteur qui est utilisée comme valeur de consigne dans une seconde boucle de régulation de la dite valeur de la $MAV_{PIR}$ en sortie.

La méthode de contrôle et régulation d'unités industrielles d'hydrogénation sélective de coupes essences oléfiniques selon l'invention nécessite donc des prélèvements d'échantillons en entrée et en sortie de l'unité, avec une fréquence de prélèvement des dits échantillons, d'un échantillon par heure, et préférentiellement un échantillon par demi-heure.

L'ensemble des échantillons d'entrée et de sortie prélevés au cours du temps forme une série.

La détermination de la MAV de chaque échantillon i d'une série fait appel à une méthode par soustraction de spectres, dite méthode delta, caractérisée par la succession d'opérations suivantes :

- on prélève une série échantillons en entrée ou en sortie de l'unité d'hydrogénation sélective à une fréquence déterminée,
- on choisit à l'intérieur de la série d'échantillons un échantillon de référence pour lequel on détermine la MAV par voie chimique,
- on obtient les spectres PIR de chaque échantillon i de la série,
- on forme la différence de spectre entre le spectre de chaque échantillon i et le spectre de l'échantillon de référence,
- on déduit le Delta_MAV de chaque échantillon à partir de la différence de spectre du dit échantillon i avec le spectre de l'échantillon de référence, en utilisant une corrélation obtenue à partir d'une base de calibration,
- on obtient la MAV de l'échantillon i à partir du Delta_MAV de l'échantillon i et de la MAV de l'échantillon de référence.

La méthode de contrôle et régulation d'unités industrielles d'hydrogénation sélective de coupes essences oléfinique selon l'invention nécessite une base de calibration composée d'au moins n échantillons prélevés sur la charge, ou sur l'effluent, ou de façon préférée, composée d'éléments pris sur la charge et d'autres éléments pris sur l'effluent. Le nombre d'échantillons n est supérieur à 20, et préférentiellement supérieur à 30.

De manière générale la base de calibration n'est pas figée, et il est possible de lui ajouter au cours du temps des éléments qui permettent d'élargir son domaine d'application.

La méthode de contrôle et régulation d'unités industrielles d'hydrogénation sélective de coupes essences oléfinique selon l'invention permet de produire des essences dont la teneur en dioléfines est optimale pour limiter à la fois la vitesse de désactivation du catalyseur d'hydrogénation sélective ainsi que le vieillissement prématuré des catalyseurs contenus dans les unités de traitement de l'essence situées en aval de l'hydrogénation sélective.

En effet, une teneur en dioléfines excessive de l'effluent pourrait conduire à une désactivation prématurée des catalyseurs utilisés dans les traitements en aval de l'hydrogénation sélective par un dépôt de polymères accru.

## DESCRIPTION DETAILLEE DE L'INVENTION

La présente invention est une méthode de contrôle et régulation des unités d'hydrogénation sélective des essences oléfiniques, telles qu'on les rencontre dans les procédés de désulfuration des essences de craquage catalytique, des essences de pyrolyse, des essences de cokéfaction ou de leurs mélanges.

De manière générale , on peut caractériser les essences aux quelles s'applique la présente invention par leur teneur en soufre comprise entre 10 ppm et 10000ppm, leur teneur en oléfines d'au moins 5 % poids, leur teneur en dioléfines d'au moins 0,1 % poids, et par leur intervalle de distillation compris entre 40°C et 300°C, et préférentiellement compris entre 40°C et 250°C.

La présente invention s'applique en fait à toute unité d'hydrogénation sélective d'essences demandant une grande précision dans le taux d'hydrogénation.

Dans le contexte des procédés de désulfuration des essences de craquage catalytique, il est essentiel de limiter l'hydrogénation aux composés de type dioléfines responsables de la formation de gommes pouvant obstruer les unités en aval, sans aller jusqu'à l'hydrogénation des mono-oléfines qui contribuent à la bonne valeur de l'indice d'octane de l'essence produite.

Le contrôle des unités d'hydrogénation sélective est actuellement réalisé au moyen d'une boucle de régulation portant sur le contrôle de la quantité d'hydrogène introduite dans l'unité. Cette quantité d'hydrogène est définie par rapport à la quantité nécessaire à l'hydrogénation des dioléfines (appelée quantité stoechiométrique) en lui ajoutant un certain excès.

Dans la présente invention le contrôle des unités d'hydrogénation sélective est fait à partir d'une mesure de la MAV en entrée et en sortie de l'unité, chacune de ces deux mesures servant à élaborer une valeur de consigne respectivement pour une boucle de régulation d'entrée et une boucle de régulation de sortie.

La première boucle de régulation ou boucle d'entrée, contrôle le débit d'hydrogène en entrée en agissant sur la quantité d'hydrogène à introduire dans l'unité à partir de la mesure de la MAV de la charge, et la seconde boucle ou boucle de sortie, contrôle la MAV de l'effluent en agissant sur la température d'entrée du réacteur d'hydrogénation sélective.

Si l'unité possède plusieurs réacteurs d'hydrogénation sélective fonctionnant en série, chacun d'entre eux, ou au moins le premier et le dernier réacteur de la série, font l'objet du système de contrôle et régulation selon la présente invention.

Dans la suite du texte on décrit de manière détaillée :

1) la méthode d'obtention de la MAV à partir du spectre PIR,
2) la boucle de régulation de la MAV d'entrée,
3) la boucle de régulation de la MAV de sortie.

1) Méthode d'obtention de la MAV à partir du spectre proche infra rouge (MAV$_{PIR}$)

La méthode d'obtention de la MAV à partir du spectre PIR, ou en abrégé modélisation PIR, consiste à corréler les spectres obtenus dans le domaine du proche infra rouge (PIR) avec une ou plusieurs propriétés d'une base d'échantillons dite base de calibration. On utilisera dans la suite indifféremment les expressions "obtenir la MAV à partir du spectre PIR" ou "modéliser la MAV par PIR".

Tout nouvel échantillon dont on cherche à déterminer la propriété P à partir de son spectre PIR est comparé à la dite base de calibration, et la propriété P n'est calculée par la corrélation que si le nouvel échantillon se trouve à l'intérieur du domaine couvert par la base de calibration.

Les corrélations entre spectres et propriétés sont développées à l'aide de méthodes statistiques d'analyse connues dans la littérature, par exemple celle définie par Martens H. Naes T. « Multivariate Calibration » Ed. John Wiley & Sons, Great Britain, 1991. Ces méthodes statistiques sont par exemple la régression linéaire multiple (MLR ou multilinear regression en anglais), la régression par composantes principales (PCR ou principal component regression en anglais), ou la méthode dite des moindres carrés partiels (PLS ou partial least squares en anglais).

La MAV des essences en sortie des unités industrielles d'hydrogénation sélective peut atteindre des valeurs très faibles qui sont généralement inférieures ou égales à 2 milligrammes/gramme.

Ces valeurs de la MAV sont équivalentes à des teneurs en dioléfines conjuguées inférieures à 0,3 % poids dans les essences.

Or la limite couramment pratiquée pour modéliser des propriétés par PIR est de 1 % poids environ.

La prédiction de la teneur en dioléfines conjuguées pour les essences en sortie d'hydrogénation sélective est donc difficile à réaliser par les techniques classiques de modélisation PIR car les bandes d'absorption des oléfines interfèrent avec celles des dioléfines. De plus, les dioléfines sont présentes en très faible teneur dans les échantillons.

En outre, les bandes d'absorption des spectres représentent surtout les hydrocarbures majoritaires présents dans les essences (paraffines, aromatiques, naphtènes, oléfines) et en très faible proportion les dioléfines conjuguées. Ainsi, les modèles classiques développés à partir des informations brutes des spectres sont très difficilement applicables à la détermination de la MAV des essences, particulièrement en sortie des unités d'hydrogénation sélective.

La présente invention décrit une méthode de traitement des spectres PIR permettant la prédiction de la MAV dans le contexte du procédé d'hydrogénation sélective des essences.

Dans le procédé d'hydrogénation sélective des essences de craquage catalytique ou thermique, les différences en composition chimique des effluents au cours du temps sont majoritairement dues à la variation de la teneur en dioléfines conjuguées et à la faible hydrogénation des oléfines.

A partir de cette constatation, au lieu de modéliser directement la MAV, la méthode de la présente invention propose la modélisation de la variation de la MAV entre un échantillon de référence et un échantillon issu du procédé en entrée ou en sortie, désigné dans la suite du texte par « échantillon i ». L'échantillon i est un effluent de MAV inconnue dont on détermine la MAV de manière séquentielle ou continue par la méthode de variation de la MAV, dite méthode "delta".

La variation de la MAV calculée par PIR est désignée comme le Delta_MAV$_{PIR}$ et se calcule comme Delta_MAV$_{PIR\,i}$ = MAV$_{référence}$ - MAV$_{PIR\,i}$.

L'échantillon de référence peut être soit un échantillon d'une série prélevée sur la charge d'entrée, soit un échantillon d'une série prélevée sur l'effluent du procédé.

La MAV de l'échantillon de référence doit être déterminée par la méthode chimique.

Si le Delta_MAV$_{PIR}$ de l'effluent i est calculé par le modèle PIR, la MAV inconnue de cet échantillon peut être calculée par différence. MAV$_{PIR\,i}$. = MAV$_{référence}$ - Delta_MAV$_{PIR\,i}$.

Les étapes permettant d'obtenir les valeurs de MAV par proche infrarouge (désigné MAV$_{PIR}$) des essences issues des unités d'hydrogénation sélective sont décrites par la suite :

- Obtention des échantillons :

Les échantillons sont des essences de craquage catalytique ou des essences de craquage thermique prélevés en entrée ou en sortie de l'unité d'hydrogénation sélective des dites essences oléfiniques. On désigne par "série" un ensemble d'échantillons prélevés en entrée ou en sortie de l'unité.

Un échantillon de la série doit être choisi comme référence. Généralement l'échantillon de référence est le premier dans l'ordre chronologique de prélèvement des échantillons de la dite série.

L'analyse PIR peut être réalisée avec ou sans échantillonnage (délocalisée, en ligne, ou par tout autre moyen de dérivation de l'échantillon). De façon préférée, l'analyse PIR sera réalisée sans échantillonnage.

Si la charge de l'unité est modifiée, il peut être utile de choisir un nouvel échantillon de référence aussi bien dans une série d'entrée que dans une série de sortie.

Cette opération doit s'effectuer une fois que l'unité est en régime stabilisé pour éviter de prélever des échantillons correspondant à des mélanges de différentes charges, comme cela peut arriver en régime transitoire.

Lorsque l'unité est en régime, la fréquence de prise d'échantillons est limitée par la durée de traitement du spectre PIR. De manière générale, une fréquence de prélèvement d'un échantillon par heure ou par demi heure est aisément praticable. Cette fréquence peut en cas de besoin être plus élevée.

- Analyse des échantillons :

L'analyse des échantillons se compose de 2 étapes principales :

o Détermination de la MAV par voie chimique pour l'échantillon de référence.
o Détermination des spectres PIR des échantillons de la série. Les spectres PIR doivent être enregistrés en mode transmission dans une gamme de longueur d'onde comprise entre 800 nm et 2500 nm. Les spectres doivent être déterminés uniquement sur des fractions liquides des essences.

- Prétraitement des spectres PIR :

Les spectres doivent être soumis aux prétraitements classiques pratiqués couramment pour la modélisation de propriétés par PIR. Ces prétraitements sont connus et décrits dans la littérature [Martens et al.]. La présente invention utilise un prétraitement qui consiste à corriger la ligne de base des spectres entre les longueurs d'onde concernées par le modèle PIR.

Après ce premier prétraitement, la différence de spectres entre l'échantillon de référence de la série et un échantillon quelconque i de la dite série est calculée selon l'équation 1.

$$A_{diff_{ref-i\,k}} = (A_{ref_k} - A_{i_k}) \qquad\qquad \text{Eq 1}$$

où :

$A_{diffref-i\,k}$ est la différence en absorbance entre l'échantillon de référence et un échantillon i à la longueur d'onde k,
$A_{ref\,k}$ est l'absorbance de l'échantillon de référence à la longueur d'onde k,
$A_{ik}$ est l'absorbance de l'échantillon i.

L'équation 1 est appliquée de k =1 à K longueurs d'onde concernées dans le modèle PIR.

L'équation 1 est appliquée à tous les échantillons i formant la série pour lesquels la MAV est inconnue.

On vérifie l'appartenance de chaque échantillon i aux échantillons formant la base de calibration.

Cette vérification d'appartenance permet de déterminer si le modèle PIR peut être appliqué à chaque échantillon i de la série étudiée. Cette vérification d'appartenance consiste en une série de tests connus de l'homme du métier.

Parmi les tests d'appartenance classiques, on peut citer le calcul de l'influence de l'échantillon i sur le modèle PIR. L'influence consiste à calculer le poids de l'échantillon i dans le modèle PIR et sa variance résiduelle. Ces tests sont du domaine publique et décrits dans la littérature [par exemple Martens et al.].

- Détermination de la MAV de l'échantillon i:

La propriété Delta_MAV$_{PIR}$ de chaque échantillon qui a franchi le test d'appartenance du paragraphe précédent peut être calculée à partir du modèle PIR établi à partir d'une base de calibration pour laquelle les différences de spectres et leurs correspondants Delta_MAV$_{PIR}$ sont connus.

La MAV$_{PIR}$ de chaque échantillon dont on connaît le Delta_MAV$_{PIR}$ peut être calculée à partir de l'équation 2 :

$$MAV_{PIR_i} = MAV_{ref} - Delta\_MAV_{PIR_i} \qquad\qquad \text{Eq. 2}$$

où:

MAV$_{PIRi}$ est la MAV de l'échantillon i de la série calculée par PIR en mg/g,

MAV$_{ref}$ est la MAV par voie chimique de l'échantillon de référence de la série en mg/g,

Delta_MAV$_{PIRi}$ est le résultat du modèle PIR pour l'échantillon i en mg/g.

Les étapes précédemment décrites sont valables pour calculer la MAV$_{PIR}$ pour le contrôle de l'unité à condition que la charge à l'entrée du procédé reste constante. Si la charge à l'entrée de l'unité change, un nouvel échantillon de référence doit être prélevé et analysé. Les données correspondantes dans les calculs des équations 1 et 2 doivent alors être modifiées en conséquence.

2) Description de la première boucle de régulation de la MAV d'entrée (B1)

La description qui suit est faite en référence à la figure 1.
Un calcul corrélatif, basé sur un modèle cinétique de la réaction, permet de déterminer, en boucle ouverte, le ratio optimal H$_2$/HC auquel la réaction doit avoir lieu. Ce calcul corrélatif utilise la valeur de la MAV d'entrée calculée à partir des échantillons successifs de la charge. Chacun des échantillons prélevés sur la charge fournit un spectre PIR duquel on déduit la MAV selon la méthode delta décrite au paragraphe précédent. Il convient de noter que ce calcul corrélatif est applicable quelque soit le modèle cinétique utilisé pour représenter la réaction chimique d'hydrogénation.
Une boucle de contrôle avancé (B1) pilote ensuite le point de consigne du débitmètre d'hydrogène, en tenant compte de la consigne de H$_2$/HC élaborée par le calcul corrélatif, et de la mesure de H$_2$/HC basée elle-même sur la mesure du débit de charge et du débit d'H$_2$.
La boucle de régulation matérialisée par le contrôleur de débit (FC), permet donc automatiquement de réguler le débit d'hydrogène de façon à se trouver à chaque instant au plus près de la valeur de consigne notée H2/HC.
La ligne C représente schématiquement l'élaboration du point de consigne H2/HC. la ligne M représente schématiquement la mesure de la teneur en dioléfines de la charge dite MAV$_{PIR}$ obtenue à partir du spectre PIR de l'échantillon considéré selon la méthode delta.
La ligne A désigne l'action de régulation automatique du débit d'hydrogène au moyen du contrôleur de débit (FC).
La présente invention fait donc appel à une première boucle de régulation ou boucle d'entrée, qui contrôle le débit d'hydrogène à introduire dans l'unité à partir de la mesure de la MAV$_{PIR}$ de la charge qui permet d'élaborer la valeur de consigne du rapport H2/HC.

3) Description de la seconde boucle de régulation de la MAV de sortie (B2).

La description qui suit est faite en référence à la figure 1.
Une boucle de contrôle avancée (B2) ajuste le point de consigne de la température d'entrée du réacteur, afin de réaliser dans les meilleures conditions le degré d'hydrogénation des dioléfines recherché.
Un modèle dynamique (calé en temps réel) entre la température d'entrée du réacteur et la MAV de l'effluent, est utilisé pour le calcul des actions de modification de la dite température d'entrée.
Ce modèle dynamique peut être vu comme une correspondance établie au fil du temps entre la température d'entrée du réacteur et la MAV$_{PIR}$ de l'effluent obtenue à partir du spectre PIR des échantillons d'effluents d'une série donnée. En s'appuyant sur cette correspondance, on peut donc transformer une consigne de teneur en dioléfines dans l'effluent en une consigne de température d'entrée du réacteur.
La ligne M représente schématiquement la mesure de la teneur en dioléfines de l'échantillon de sortie, ou MAV$_{PIR}$ de l'effluent, obtenue à partir de son spectre PIR selon la méthode delta exposée au paragraphe précédent. la ligne A représente schématiquement l'action de régulation automatique effectuée par le contrôleur de température (TC) à partir de la mesure M et de la valeur de consigne de la température d'entrée du réacteur calculée à partir d'un modèle dynamique.
La méthode de contrôle et régulation d'unités industrielles d'hydrogénation sélective de coupes essences oléfinique selon l'invention fait donc appel à une seconde boucle de régulation ou boucle de sortie qui contrôle la température d'entrée du réacteur à partir d'une consigne élaborée à partir de la MAV$_{PIR}$ de l'effluent, la dite MAV$_{PIR}$ étant déterminée sur une série d'échantillons de sortie de l'unité à partir de leur spectre PIR selon la méthode delta.

## EXEMPLE SELON L'INVENTION

On traite dans cet exemple trois essences oléfiniques dont les caractéristiques sont données dans le tableau 1 ci dessous, dans une unité d'hydrogénation sélective travaillant avec un catalyseur à base de nickel et de molybdène.

Les valeurs de la température en entrée de réacteur et du rapport adimensionnel H2/ HC, (HC désignant la charge), sont ici données à titre purement illustratif et ne limitent aucunement la portée de l'invention qui s'applique à toute unité d'hydrogénation sélective d'essences oléfiniques.

Trois séries d'échantillons, correspondant à chacune des charges traitées, ont été prélevées en sortie de l'unité d'hydrogénation sélective d'essence oléfinique à une fréquence d'un échantillon toute les demi heure.

Ces effluents correspondent aux différentes conditions opératoires de l'unité d'hydrogénation sélective reprises dans le tableau 2.

Les valeurs de $MAV_{PIR}$ des échantillons ont été calculées via la méthode « $Delta\_MAV_{PIR}$ ».

L'unité disposant d'un analyseur en ligne, la mesure PIR a pu être réalisée en temps réel et ainsi éviter le dépassement de la MAV cible (2 mg/g) en sortie de l'unité grâce aux deux boucles de régulation proposées dans cette invention, telles que décrites dans la description détaillée.

Le tableau 2 ci dessous compare les valeurs de la $MAV_{PIR}$ déterminée selon la méthode delta sur les 3 séries d'échantillons, avec les valeurs de la MAV chimique. L'accord entre les 2 séries de valeur est remarquable et permet un pilotage très fin de l'unité au moyen des 2 boucles de régulation décrites dans le texte.

Tableau 1 : Caractéristiques globales des charges de chaque série.

|  |  | Série 1 | Série 2 | Série 3 |
|---|---|---|---|---|
| densité à 15°C | g/cm$^3$ | 0,779 | 0,738 | 0,772 |
| Soufre | ppm poids | 2580 | 1260 | 3480 |
| Distillation simulée 5% | °C | 23 | 23 | 23 |
| Distillation simulée 50% | °C | 140 | 92 | 126 |
| Distillation simulée 95 % | °C | 217 | 183 | 233 |
| MAV | mg/g | 13.0 | 9.9 | 17 |
| Paraffines | % poids | 22 | 31 | 22 |
| Oléfines | % poids | 31 | 34 | 36 |
| Naphtènes | % poids | 6 | 8 | 6 |
| Aromatiques | % poids | 41 | 27 | 36 |

Tableau 2: valeurs comparées de la MAV chimique et selon la méthode delta PIR pour différentes conditions opératoires de l'unité d'hydrogénation sélective

|  | N° Echantillon de la série | $MAV_{chimique}$ (mg/g) | $MAV_{PIR}$ (mg/g) | Température (°C) | H2/HC |
|---|---|---|---|---|---|
| Série 1 | 1 | 1,8 | 1,9 | 170 | 25 |
|  | 2 | 1,5 | 1,7 | 170 | 25 |
|  | 3 | 5,8 | 6,2 | 170 | 20 |
| Série 2 | 1 | 5,4 | 5,4 | 150 | 25 |
|  | 2 | 2,8 | 3,2 | 150 | 20 |
|  | 3 | 2,5 | 2,6 | 150 | 25 |
|  | 4 | 6,7 | 6,6 | 140 | 25 |
|  | 5 | 3,5 | 3,7 | 150 | 25 |
|  | 6 | 0,7 | 0,6 | 170 | 25 |
|  | 7 | 0,9 | 0,9 | 150 | 25 |
|  | 8 | 0,5 | 0,9 | 150 | 25 |
| Série 3 | 1 | 5,9 | 6,4 | 140 | 20 |
|  | 2 | 3,7 | 3,5 | 150 | 20 |
|  | 3 | 7,6 | 8,2 | 140 | 20 |
|  | 4 | 3,5 | 3,2 | 170 | 20 |
|  | 5 | 8,6 | 8,5 | 140 | 20 |

**Revendications**

1. Méthode de contrôle et régulation d'unités industrielles d'hydrogénation sélective de coupes essences oléfiniques consistant en la succession d'étapes suivantes:

   - on prélève un échantillon en entrée et en sortie de la dite unité à une fréquence déterminée,
   - on analyse les dits échantillons par leur spectre PIR en absorption dans une gamme de longueur d'onde comprise entre 800 nm et 2500 nm,
   - on détermine la $MAV_{PIR}$ des échantillons d'entrée et de sortie par un traitement des spectres PIR faisant appel à une base de calibration et un prétraitement spécifique,
   - on détermine à partir de la $MAV_{PIR}$ de l'échantillon d'entrée et du débit de charge, la quantité d'hydrogène à introduire dans l'unité qui est utilisée comme valeur de consigne dans une première boucle de régulation de la dite valeur de la $MAV_{PIR}$ en entrée,
   - on détermine à partir de la $MAV_{PIR}$ de l'échantillon de sortie et d'un modèle dynamique la température d'entrée optimale dans le réacteur qui est utilisée comme valeur de consigne dans une seconde boucle de régulation de la dite valeur de la $MAV_{PIR}$ en sortie,

   et dans laquelle la détermination de la MAV de chaque échantillon i d'une série fait appel à une méthode par soustraction de spectres, dite méthode delta, **caractérisée par** la succession d'opérations suivantes :

   - on prélève une série échantillons en entrée ou en sortie de l'unité d'hydrogénation sélective à une fréquence déterminée,
   - on choisit à l'intérieur de la série d'échantillons un échantillon de référence pour lequel on détermine la MAV par voie chimique,
   - on obtient les spectres PIR de chaque échantillon i de la série,
   - on forme la différence de spectre entre le spectre de chaque échantillon i et le spectre de l'échantillon de référence,
   - on déduit le Delta_MAV de chaque échantillon à partir de la différence de spectre du dit échantillon i avec le spectre de l'échantillon de référence en utilisant une corrélation obtenue à partir d'une base de calibration,

   on obtient la MAV de l'échantillon i à partir du Delta_MAV de l'échantillon i et de la MAV de l'échantillon de référence.

2. Méthode de contrôle et régulation d'unités industrielles d'hydrogénation sélective de coupes essences oléfiniques selon la revendication 1 dans laquelle la fréquence de prélèvement des échantillons d'entrée et de sortie est d'un échantillon par heure, et préférentiellement d'un échantillon par demi-heure.

3. Méthode de contrôle et régulation d'unités industrielles d'hydrogénation sélective de coupes essences oléfinique selon l'une quelconque des revendications 1 ou 2 dans laquelle la base de calibration est composée d'au moins n échantillons prélevés à la fois sur la charge et sur l'effluent, n étant supérieur à 20.

4. Méthode de contrôle et régulation d'unités industrielles d'hydrogénation sélective de coupes essences oléfinique selon l'une quelconque des revendications 1 ou 2 dans laquelle la base de calibration est composée d'au moins n échantillons prélevés à la fois sur la charge et sur l'effluent, n étant supérieur à 30.

5. Méthode de contrôle et régulation d'unités industrielles d'hydrogénation sélective de coupes essences oléfinique selon l'une quelconque des revendications 1 à 4 dans laquelle la boucle de régulation d'entrée utilise un calcul corrélatif, basé sur un modèle cinétique de la réaction, permettant de déterminer en boucle ouverte, le ratio optimal $H_2/HC$ auquel la réaction doit avoir lieu, le dit calcul corrélatif utilisant la valeur de la MAV d'entrée calculée sur une série d'échantillons de la charge à partir de leur spectre PIR selon la méthode delta .

6. Méthode de contrôle et régulation d'unités industrielles d'hydrogénation sélective de coupes essences oléfinique selon l'une quelconque des revendications 1 à 4 dans laquelle la boucle de régulation de sortie utilise comme point de consigne la température d'entrée du réacteur et un modèle dynamique calé en temps réel à partir de la température d'entrée du réacteur et de la MAV de l'effluent, la dite MAV étant déterminée sur une série d'échantillons de sortie de l'unité à partir de leur spectre PIR selon la méthode delta.

7. Méthode de contrôle et régulation d'unités industrielles d'hydrogénation sélective de coupes essences oléfinique selon l'une quelconque des revendications 1 à 6 dans laquelle la valeur de la MAV des essences en sortie desdites unités industrielle est inférieure ou égale à 2 milligramme par gramme

**Claims**

1. A method for controlling and regulating industrial units for the selective hydrogenation of olefinic gasoline cuts, consisting of the following steps in succession:

   • removing a sample from the inlet and the outlet of said unit at a predetermined frequency;
   • analyzing said samples by their absorption NIR spectrum in a wavelength range 2008 in the range 800 nm to 2500 nm;
   • determining the $MAV_{NIR}$ of the inlet and outlet samples by processing the NIR spectra employing a calibration base and specific pre-processing;
   • determining, from the $MAV_{NIR}$ of the inlet sample and the flow rate of the feed, the quantity of hydrogen to be introduced into the unit, which is used as a reference input in a first loop for regulating said inlet $MAV_{NIR}$ value;
   • determining, from the $MAV_{NIR}$ of the outlet sample and a dynamic model, the optimal inlet temperature in the reactor, which is used as a reference input in a second loop for regulating said outlet $MAV_{NIR}$;

   and in which determining the MAV of each sample i of a series employs a spectrum subtraction method, termed a delta method, **characterized by** the following operations in succession:

   • removing a series of samples from the inlet or outlet of the selective hydrogenation unit at a predetermined frequency;
   • selecting, from the series of samples, a reference sample the MAV of which is determined chemically;
   • obtaining the NIR spectra of each sample i of the series;
   • producing the difference spectrum between the spectrum of each sample i and the spectrum of the reference sample;
   • deducing the delta_MAV of each sample from the difference between the spectrum of said sample i and the spectrum of the reference sample using a correlation obtained from a calibration base;
   • obtaining the MAV of the sample i from the delta_MAV of sample i and the MAV of the reference sample.

2. A method for controlling and regulating industrial units for the selective hydrogenation of olefinic gasoline cuts according to claim 1, in which the frequency at which the inlet and outlet samples are removed is one sample per hour, preferably one sample per half-hour.

3. A method for controlling and regulating industrial units for the selective hydrogenation of olefinic gasoline cuts according to claim 1 or claim 2, in which the calibration base is composed of at least n samples removed from both the feed and the effluent, n being more than 20.

4. A method for controlling and regulating industrial units for the selective hydrogenation of olefinic gasoline cuts according to claim 1 or claim 2, in which the calibration base is composed of at least n samples removed from both the feed and from the effluent, n being more than 30.

5. A method for controlling and regulating industrial units for the selective hydrogenation of olefinic gasoline cuts according to any one of claims 1 to 4, in which the inlet regulation loop employs a correlative calculation based on a kinetic model of the reaction, allowing the optimal $H_2$/HC ratio at which the reaction should take place to be determined under open loop conditions, said correlative calculation employing the inlet MAV value calculated for a series of samples of the feed derived from their NIR spectrum using the delta method.

6. A method for controlling and regulating industrial units for the selective hydrogenation of olefinic gasoline cuts according to any one of claims 1 to 4, in which the outlet regulation loop uses, as a reference input, the inlet temperature of the reactor and a fixed real-time dynamic model derived from the inlet temperature of the reactor and the MAV of the effluent, said MAV being determined for a series of outlet samples from the unit derived from their NIR spectrum using the delta method.

7. A method for controlling and regulating industrial units for the selective hydrogenation of olefinic gasoline cuts according to any one of claims 1 to 6, in which the value of the MAV of the gasolines at the outlet from said industrial units is 2 milligrams per gram or less.

**Patentansprüche**

1.  Verfahren zur Steuerung und Regulierung von industriellen Einheiten zur selektiven Hydrierung von olefinischen Benzinschnitten, das aus der Abfolge der folgenden Schritte besteht:

    - Entnehmen einer Probe am Einlass und am Auslass der Einheit in einer bestimmten Frequenz,
    - Analysieren der Proben mittels ihres NIR-Spektrums durch Absorption in einem Wellenlängenbereich zwischen 800 nm und 2.500 nm,
    - Bestimmen des $MAV_{NIR}$ der Einlass- und Auslassproben durch eine Behandlung der NIR-Spektren unter Verwendung einer Kalibrierungsbasis und einer spezifischen Vorbehandlung,
    - Bestimmen, aus dem $MAV_{NIR}$ der Einlassprobe und der Durchflussmenge der Beschickung, der Menge an Wasserstoff, die in die Einheit einzuführen ist, die als Sollwert in einer ersten Regulierungsschleife des $MAV_{NIR}$ am Einlass verwendet wird,
    - Bestimmen, aus dem $MAV_{NIR}$ der Auslassprobe und aus einem dynamischen Modell, der optimalen Einlasstemperatur in den Reaktor, die als Sollwert in einer zweiten Regulierungsschleife des $MAV_{NIR}$ am Auslass verwendet wird,

    und worin die Bestimmung des MAV jeder Probe i einer Reihe unter Verwendung eines Verfahrens der spektralen Subtraktion, des so genannten Delta-Verfahrens, durchgeführt wird, das durch die Abfolge der folgenden Vorgänge **gekennzeichnet** ist:

    - Entnehmen einer Reihe von Proben am Einlass und am Auslass der Einheit zur selektiven Hydrierung mit einer bestimmten Frequenz,
    - Auswählen einer Referenzprobe aus der Reihe von Proben, für die der MAV auf chemischem Weg bestimmt wird,
    - Erhalten der NIR-Spektren jeder Probe i der Reihe,
    - Bilden der Spektrendifferenz zwischen dem Spektrum jeder Probe i und dem Spektrum der Referenzprobe,
    - Ableiten der Delta_MAV jeder Probe aus der Spektrendifferenz der Probe i mit dem Spektrum der Referenzprobe unter Verwendung einer Korrelation, die aus einer Kalibrierungsbasis erhalten wird,

    Erhalten des MAV der Probe i aus der Deita_MAV der Probe i und des MAV der Referenzprobe.

2.  Verfahren zur Steuerung und Regulierung von industriellen Einheiten zur selektiven Hydrierung von olefinischen Benzinschnitten nach Anspruch 1, worin die Entnahmefrequenz von Einlass- und Auslassproben eine Probe pro Stunde, und vorzugsweise eine Probe pro halber Stunde ist.

3.  Verfahren zur Steuerung und Regulierung von industriellen Einheiten zur selektiven Hydrierung von olefinischen Benzinschnitten nach einem der Ansprüche 1 oder 2, worin die Kalibrierungsbasis aus mindestens n Proben besteht, die gleichzeitig aus der Beschickung und aus dem Abfluss entnommen werden, wobei n größer als 20 ist.

4.  Verfahren zur Steuerung und Regulierung von industriellen Einheiten zur selektiven Hydrierung von olefinischen Benzinschnitten nach einem der Ansprüche 1 oder 2, worin die Kalibrierungsbasis aus mindestens n Proben besteht, die gleichzeitig aus der Beschickung und aus dem Abfluss entnommen werden, wobei n größer als 30 ist.

5.  Verfahren zur Steuerung und Regulierung von industriellen Einheiten zur selektiven Hydrierung olefinischer Benzinschnitte nach einem der Ansprüche 1 bis 4, worin die Einlassregulierungsschleife eine korrelative Berechnung verwendet, die auf einem kinetischen Modell der Reaktion basiert, die es ermöglicht, in offener Schleife das optimale $H_2$/HC-Verhältnis zu bestimmen, bei dem die Reaktion stattfinden soll, wobei die korrelative Berechnung des MAV am Einlass verwendet wird, der anhand einer Reihe von Proben der Beschickung aus deren NIR-Spektrum gemäß dem Delta-Verfahren berechnet wird.

6.  Verfahren zur Steuerung und Regelung industrieller Einheiten zur selektiven Hydrierung von olefinischen Benzinschnitten nach einem der Ansprüche 1 bis 4, worin die Auslassregulierungsschleife als Sollwert die Einlasstemperatur des Reaktors und ein dynamisches Modell, das auf Echtzeit eingestellt ist, aus der Einlasstemperatur des Reaktors und dem MAV des Abflusses verwendet, wobei der MAV aus einer Reihe von Auslassproben der Einheit aus deren NIR-Spektrum gemäß dem Delta-Verfahren bestimmt wird.

7.  Verfahren zur Steuerung und Regulierung von industriellen Einheiten zur selektiven Hydrierung von olefinischen Benzinschnitten nach einem der Ansprüche 1 bis 6, worin der MAV des Benzins am Auslass der industriellen Einheiten kleiner oder gleich 2 Milligramm pro Gramm ist.

Figure 1

**EP 1 783 480 B1**